(19) Europäisches Patentamt

European Patent Office

Office européen des brevets

(11) **EP 1 110 569 B1**

(12) **EUROPEAN PATENT SPECIFICATION**

(45) Date of publication and mention
of the grant of the patent:
**19.07.2006 Bulletin 2006/29**

(51) Int Cl.:
*A61M 5/145* (2006.01)

(21) Application number: **00311194.5**

(22) Date of filing: **14.12.2000**

(54) **Syringe pump**

Spritzenpumpe

Pompe seringue

(84) Designated Contracting States:
**AT BE CH CY DE DK ES FI FR GB GR IE IT LI LU MC NL PT SE TR**

(30) Priority: **24.12.1999 JP 36702599**

(43) Date of publication of application:
**27.06.2001 Bulletin 2001/26**

(60) Divisional application:
**06005799.9 / 1 679 091**

(73) Proprietor: **TERUMO KABUSHIKI KAISHA
Tokyo 151 (JP)**

(72) Inventors:
• **Watanabe, Takashi
Fuji-shi,
Shizuoka-ken (JP)**
• **Nakada, Narukuni
Fuji-shi,
Shizuoka-ken (JP)**

(74) Representative: **Harrison, David Christopher et al
Mewburn Ellis LLP
York House
23 Kingsway
London WC2B 6HP (GB)**

(56) References cited:
**EP-A- 0 314 880        EP-A- 0 514 907
EP-A- 0 916 353        WO-A-94/08647
WO-A-96/25963        JP-A- 9 122 237
US-A- 5 425 716**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

FIELD OF THE INVENTION

The present invention relates to a syringe pump in which one of various types of different syringes is detachably held in an immobile state, the thumb rest (or piston rod) of the syringe is set in the immobile state with respect to a slider means, and the slider means is driven by a predetermined amount, thereby feeding the content of the syringe accurately and, more particularly, to a syringe pump in which most of its operation is automated and disengagement of the syringe during operation can be detected.

BACKGROUND OF THE INVENTION

A syringe pump mainly aims at supply of nutrition, blood transfusion, and injection of a liquid drug such as chemotherapeutics, narcotic, and the like at high precision in an intensive care unit (ICU), coronary care unit (CCU), or neonatal intensive care unit (NICU), and is often used since liquid flow rate control and the like can be controlled better than in pumps of other types.

The syringe pump is used in the following manner. A syringe containing a liquid drug is set in a syringe pump in an immobile state by using the clamp of the syringe pump, and the thumb rest of the syringe is set on the slider. The slider is then driven to feed the content in the syringe accurately upon piston motion of the thumb rest (or the piston rod). The syringe pump is often used since it has a simple structure and comparatively high precision.

In liquid drug injection performed in this manner, if the syringe is used with the flange of its thumb rest not being fixed to the slider, the liquid drug is not fed from the syringe. When the syringe is connected to a closed circuit, a negative pressure that moves the thumb rest to the injection side is generated. In this case, if the thumb rest is not set in the immobile state, a liquid drug in an amount exceeding the preset amount is sometimes injected.

Many syringe pumps have a function of detecting whether the flange of the thumb rest of the syringe is set on the slider portion, which fixes the flange of the thumb rest (or the piston rod) for the syringe, in the immobile state, or is disengaged from the slider portion, and enables injection only when the flange of the thumb rest of the syringe is set on the slider portion in the immobile state, and prohibits injection when it is not.

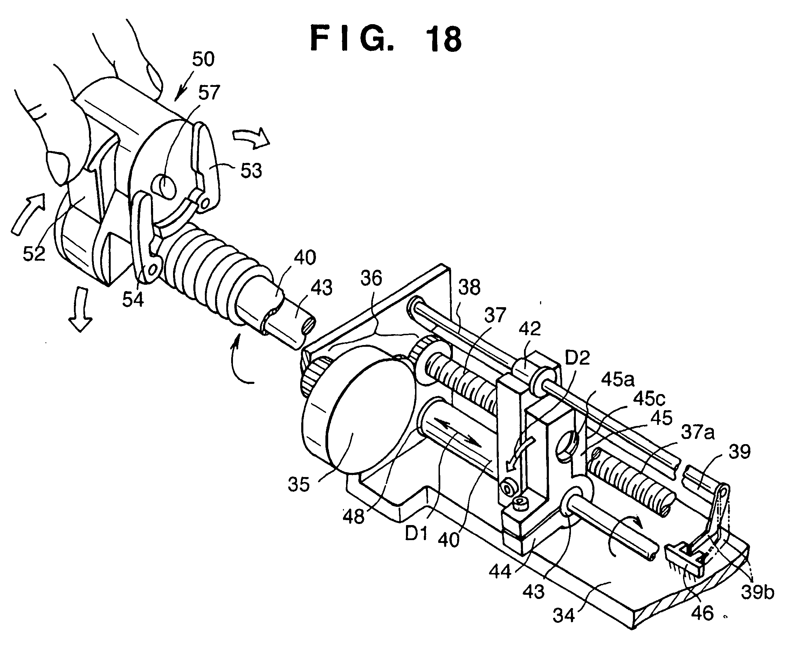

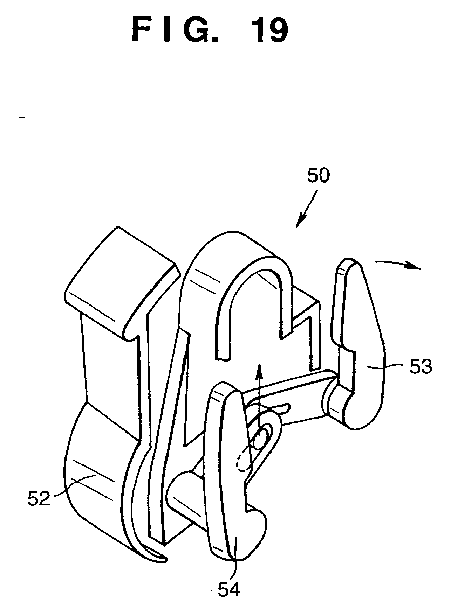

A syringe pump having the above function is disclosed in, e.g., Japanese Patent Laid-Open No. 9-122237. The arrangement of this syringe pump will be described with reference to Fig. 18 as the perspective view of the outer appearance of a slider mechanism and Fig. 19 as the perspective view of the outer appearance for explaining its operation. When the main body of syringe (not shown) is set on the main body of the syringe pump in the immobile state, the flange (not shown) of the thumb rest of the syringe is set on a slider assembly 50 driven in directions of arrows in Fig. 18. The slider assembly 50 is moved reciprocally, and simultaneously connected and fixed to a pipe shaft serving as a hollow shaft with respect to a slider feed mechanism, and to the end of an inner clutch shaft serving as a clutch shaft. When a clutch lever 52 of the slider assembly 50 is manually operated, the flange of the thumb rest can be mounted and removed easily.

In the above arrangement, as shown in Fig. 19, when the user grips the clutch lever 52 in order to set the syringe, a pair of right and left hooks 53 and 54 open in synchronism with each other, so that one of the flanges with different sizes of thumb rests can be placed.

A base 34 serving as the base portion is integrally formed with right and left wall surfaces (one of them is not shown), so that a lead screw 37 with one end to which the gear of a gear train 36 is fixed, a clutch shaft 43, and a guide shaft 38 are supported in a space portion sandwiched by the right and left wall surfaces. The lead screw 37 is pivotally supported by a bearing.

A pipe shaft 40 movable through a guide bush 48 in the directions of arrows D1 is coaxially, slidably guided on the clutch shaft 43. The guide shaft 38 is fixed to the base 34 in the immobile state. A block 41 for fixing a polyacetal resin rotation-preventive resin bush 42 in which the guide shaft 38 is inserted is fixed to the end of the pipe shaft 40. A nut holder 44 for fixing a half nut member 45, always biased in the direction of a blank arrow D2 and having a tooth portion 45a always meshing with a tooth portion 37a of the lead screw 37, is fixed to the clutch shaft 43. The nut holder 44 is disengaged and set in the free state only when the clutch shaft 43 is pivoted in a direction opposite to the arrow D2, so that it can freely move in the directions of arrows D1.

A clutch sensor plate 39 serving as the dog means of a sensor is pivotally, axially supported by the guide shaft 38. When the user grips the clutch lever 52 of the slider assembly 50, the half nut member 45 is rotated through the pipe shaft 40, and consequently the ridge portion 37a of the lead screw 37 and the ridge portion 45a of the half nut member 45 are disengaged from each other. When the half nut member 45 is rotated in this manner, the clutch sensor plate 39, biased by a biasing means (not shown) such as a coil spring, a leaf spring, and the like so its slide surface 39c always abuts against a slide surface 45c of the half nut member 45, is rotated simultaneously. When the clutch sensor plate 39 rotates, light is transmitted to the sensor serving as a photosensor (transmission type) 46 fixed inside the upper cover and normally light-blocked by a lever portion 39b at one end of the clutch sensor plate 39.

When this light transmission is detected, an alarm indicating that the syringe is not mounted is displayed. When a motor 35 is driven in order to move the flange of the thumb rest, the lead screw 37 is rotationally driven, and the nut holder 44 fixing the half nut member 45 which meshes with the lead screw 37 is moved. Hence, the pipe shaft 40 is moved to move the slider assembly 50. At this time, the lever portion 39b at one end of the clutch sensor plate 39 is set in the state indicated by a solid line in Fig. 18 where it blocks light to the photosensor (transmission type) 46. A contact pin 57 biased such that its one end always projects by the operation of a torsion spring is provided at a portion on a side surface of the base of the slider assembly 50. The starting state can be set only when the flange of the thumb rest abuts against the contact pin 57 to move it inward.

In a syringe disengagement detection apparatus of European Patent No. 514907B1, a technique is disclosed which enables detection of disengagement of the syringe during operation by optically detecting the motion of a component corresponding to the contact pin provided to the slider assembly 50 described above.

SUMMARY OF THE INVENTION

In the syringe pump described above, the half nut member is completely fixed to one end of the clutch shaft, and is positioned in a meshing state where it meshes with the thread portion of the lead screw, and in a disengaging state where it is disengaged from it. Presence/absence of the thumb rest is detected only at the start of the syringe pump.

The apparatus of European Patent No. 514907B1 can detect disengagement of the flange of the thumb rest during operation as well. According to this disclosure, however, since the portion for fixing the thumb rest and the detecting portion for detecting the flange of the thumb rest are at the same position, the flange of the thumb rest can be easily disengaged from the slider assembly.

Also, according to this disclosure, the sensor incorporated in the slider assembly electrically detects the presence/absence of the syringe, and the obtained electric signal is connected to the main body of the apparatus through an electric wire or flexible board. Therefore, the apparatus tends to be affected by the electric noise produced by this electrical connection.

The present invention has been made in consideration of the problems described above, and has as its object to provide a syringe pump with which whether the flange of the thumb rest of a syringe is set on the slider assembly of the syringe pump can be detected regardless of whether the pump is in operation or stopped and the flange of the thumb rest can be reliably fixed to the slider assembly, and which is not affected by electric noise.

In order to solve the above problems and achieve the above objects, according to the present invention, there is provided a syringe pump for detachably holding a syringe main body (a syringe cylinder) on an apparatus main body (a main body of a syringe pump) in an immobile state, setting a flange of a thumb rest of the syringe on slider means in an immobile state, and driving the slider means, thereby accurately feeding out a content in the syringe, characterized in that

the slider means comprises

a lead screw axially supported by a base portion so as to be driven rotationally,

a hollow shaft provided to the base portion to be extendable in a longitudinal direction of the syringe,

a clutch shaft rotatably provided in a hollow portion of the hollow shaft,

a half nut member provided to one end of the clutch shaft and positioned in a meshing state to mesh with a thread portion of the lead screw and in a disengaged state disengaged from the thread portion,

a detector with dog means to abut against the half nut member and block light to a sensor fixed to the base portion in order to detect that the half nut member is in the meshing state,

a movable base portion fixed to the other end of the hollow shaft,

the movable base portion being comprised of a pair of hooks for setting the thumb rest in the immobile state and converting means for converting an abutting state against the thumb rest of the flange into rotation of the clutch shaft through a contact pin, and

a power transmission mechanism for transmitting a moving amount of the contact pin to the half nut member before the detector detects that the half nut member is in the meshing state upon movement of the dog means,

wherein the detector can detect that the flange of the thumb rest has disengaged from the hooks.

Other features and advantages of the present invention will be apparent from the following description taken in conjunction with the accompanying drawings, in which like reference characters designate the same or similar parts throughout the figures thereof.

BRIEF DESCRIPTION OF THE DRAWINGS

Fig. 1 is a partially cutaway perspective view of the outer appearance of a slider mechanism to show a base 34 serving as a base portion;

Fig. 2 is a perspective view of the outer appearance of a slider assembly 50 from which a syringe pump cover is removed;

Fig. 3 is a sectional view taken along the line of arrows Y - Y of Fig. 1;

Fig. 4 is a side view of Fig. 2;

Fig. 5 is a sectional view taken along the line of arrows X - X of Fig. 4 for explaining the operation of a contact pin 1;

Fig. 6 is a sectional view taken along the line of arrows X - X of Fig. 1 for showing a meshing state during operation;

Fig. 7 is a sectional view taken along the line of arrows X - X of Fig. 1 for showing an inoperative state;

Fig. 8 is a table showing operation specifications;

Fig. 9 is a perspective view of the outer appearance of the syringe pump;

Fig. 10 is a plan view of an operation panel;

Fig. 11 is a block diagram of the syringe pump;

Figs. 12 and 13 are flow charts for explaining the operation of the first embodiment;

Figs. 14 and 15 are flow charts for explaining the operation of the second embodiment;

Figs. 16 and 17 are flow charts for explaining the operation of the third embodiment;

Fig. 18 is a perspective view of the outer appearance of a conventional slider mechanism; and

Fig. 19 is a perspective view of the outer appearance of a conventional slider assembly 50.

## DETAILED DESCRIPTION OF THE PREFERRED EMBODIMENTS

The preferred embodiments of the present invention will be described by way of a mechanism commonly used in the respective embodiments with reference to the accompanying drawings.

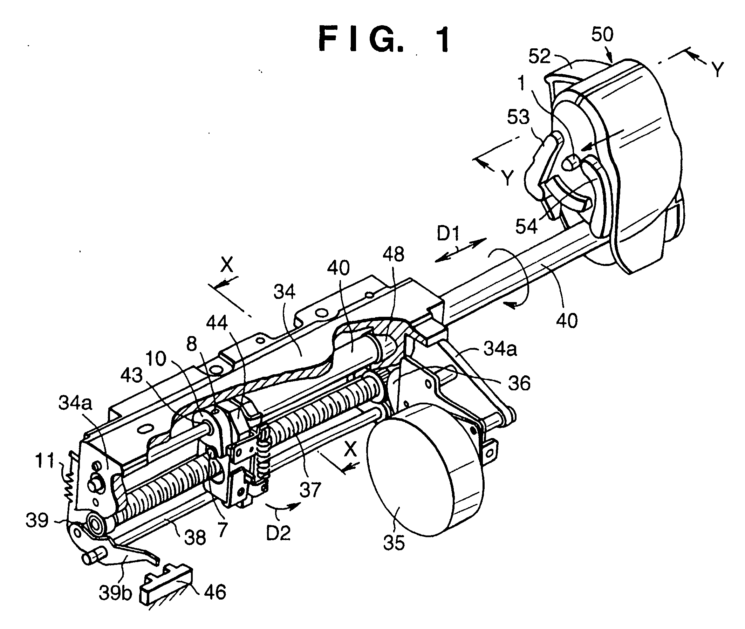

Fig. 1 is a partially cutaway perspective view of the outer appearance of a slider mechanism to show a base 34 serving as a base portion.

Referring to Fig. 1, when the main body of syringe (not shown) is set on the main body of the syringe pump in the immobile state, the thumb rest is set on a slider assembly 50 driven in directions of arrows in Fig. 1.

The slider assembly 50 meshes with a lead screw as will be described later so that it is driven to move. Simultaneously, the slider assembly 50 is connected and fixed to a pipe shaft 40 serving as a hollow shaft with respect to a slider feed mechanism, and to the end of a clutch shaft 43. When a clutch lever 52 of the slider assembly 50 is manually operated, hooks 53 and 54 are opened and closed, so that the flange (not shown) of the thumb rest can be mounted and removed easily.

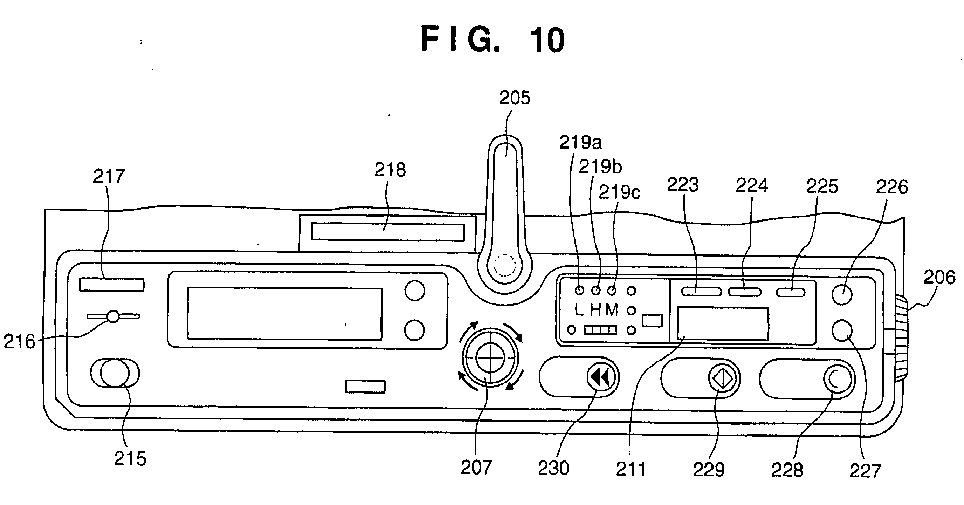

In the above arrangement, when the user grips the clutch lever 52 in order to set the syringe, the pair of left and right hooks 53 and 54 are opened in synchronism with each other, as has been described with reference to Fig. 10 as well, so that one of the flanges with different sizes of thumb rests can be held and placed on the slider assembly 50 in the substantially immobile state.

The base 34 serving as the base portion is integrally formed with right and left wall surfaces 34a by aluminum die casting, so that a lead screw 37 with one end to which the gear of a gear train 36 is fixed, the clutch shaft 43, and a guide shaft 38 also serving as the pivot fulcrum of a dog means are supported in a space portion sandwiched by the right and left wall surfaces 34a. The lead screw 37 is pivotally supported by a bearing.

The pipe shaft 40 movable through a guide bush 48 in directions of arrows D1 is coaxially, slidably guided on the clutch shaft 43. The guide shaft 38 is fixed to the base 34 in the immobile state. A block for fixing a polyacetal resin rotation-preventive bush in which the guide shaft 38 is inserted is fixed to the end of the pipe shaft 40.

A nut holder 44 for fixing a half nut member 45, always biased in the direction of an arrow D2 and having a tooth portion 45a always meshing with a tooth portion 37a of the lead screw 37, is provided to the clutch shaft 43, as will be described later. The nut holder 44 is disengaged and set in the free state only when the clutch shaft 43 is pivoted in a direction opposite to the arrow D2, so that it can freely move in the directions of arrows D1 to cover substantially the entire length of the syringe.

A clutch sensor plate 39 serving as a dog means is pivotally, axially supported by the guide shaft 38. When the user grips the clutch lever 52 of the slider assembly 50, the half nut member 45 is rotated through the pipe shaft 40, as will be described later, and consequently the ridge portion 37a of the lead screw 37 and the ridge portion 45a of the half nut member 45 are disengaged from each other.

When the half nut member 45 is rotated in this manner, the clutch sensor plate 39, biased by a biasing means (not shown) so its slide surface 39c always abuts against a slide surface 45c of the half nut member 45, is rotated simultaneously. When the clutch sensor plate 39 is rotated, light is transmitted to a sensor serving as a photosensor (transmission type) 46 fixed inside the upper cover and normally light-blocked by a lever portion 39b at one end of the clutch sensor plate 39.

When this light transmission is detected, an alarm indicating that the syringe is not mounted is displayed.

When a motor 35 is driven in order to move the flange of the thumb rest, the lead screw 37 is rotationally driven, and the nut holder 44 fixing the half nut member 45 meshing with the lead screw 37 is moved. Hence, the pipe shaft 40 is moved to move the slider assembly 50. At this time, the lever portion 39b at one end of the clutch sensor plate 39 blocks light to the photosensor (transmission type) 46. A contact pin (contact member) 1 biased by a torsion spring such that its one end always projects is provided at a portion on a side surface of the base of the slider assembly 50. The starting state by the user described above can be set only when the flange of the thumb rest abuts against the contact pin 1 to move it inward.

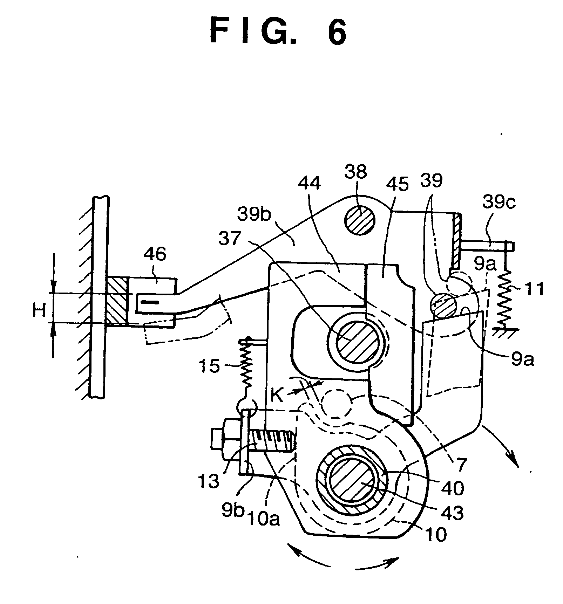

A further explanation will be made with reference to Fig. 6, which is a sectional view taken along the line of arrows X - X of Fig. 1. A power transmission mechanism is comprised of an operation member 10 fixed to one end of the clutch shaft 43 and having a projection 10a, the half nut member 44 pivotally provided to the clutch shaft 43, a stud 7 (indicated by a broken line) fixed to the side surface of a half nut member 44 to be away from the projection 10a of the operation member 10 by a predetermined gap K, and an intermediate member 9 obtained by integrally forming an abutting portion 9a, serving as a dog means pivotally provided to the base 34 and having one end abutting against the shaft 39, a hole portion pivotally supported by the clutch shaft 43, and a portion 9b provided with a spring 15 serving as a biasing means to bias the operation member 10 to separate from the stud 7 by a distance corresponding to the clearance K described above.

In the above arrangement, referring to Fig. 6, the dog portion 39b of the dog means is located at the position indicated by a solid line due to the operation of a tensile spring 11 serving as a biasing means. In the operative state wherein the lead screw 37 meshes with the half nut member 45, the dog portion 39b blocks light to the optical photosensor 46.

As will be described later, when the abutting state of the flange of the thumb rest is canceled, the contact pin 1 projects, and the clutch shaft 43 initially pivots only slightly, then the abutting surface 10a of the operation member 10 pivots in the direction of arrows from the abutting state with respect to a screw 13 against the tensile force of the spring 15, and pivots by a distance corresponding to the clearance K. As a result, the abutting portion 9a of the intermediate member 9 pivots to the position indicated by a broken line to move the shaft 39 of the dog means to the position indicated by a broken line in Fig. 6. Consequently, the dog portion 39b moves for a distance H to cancel the light-blocking state of the photosensor 46.

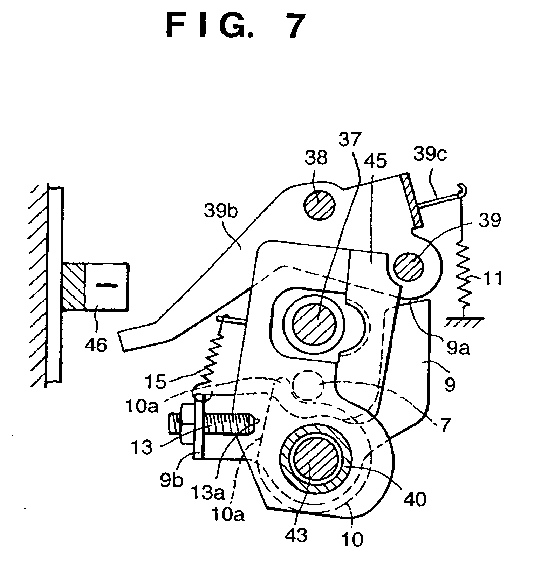

In Fig. 7, when the user grips the clutch lever 52 to further pivot the operation member 10 to the position indicated by a broken line, the operation member 10 presses the stud 7 to completely disengage the half nut member 45.

As the flange of the thumb rest is disengaged, the contact pin 1 projects and the clutch shaft 43 initially pivots only slightly. This pivot state can be detected in the above manner.

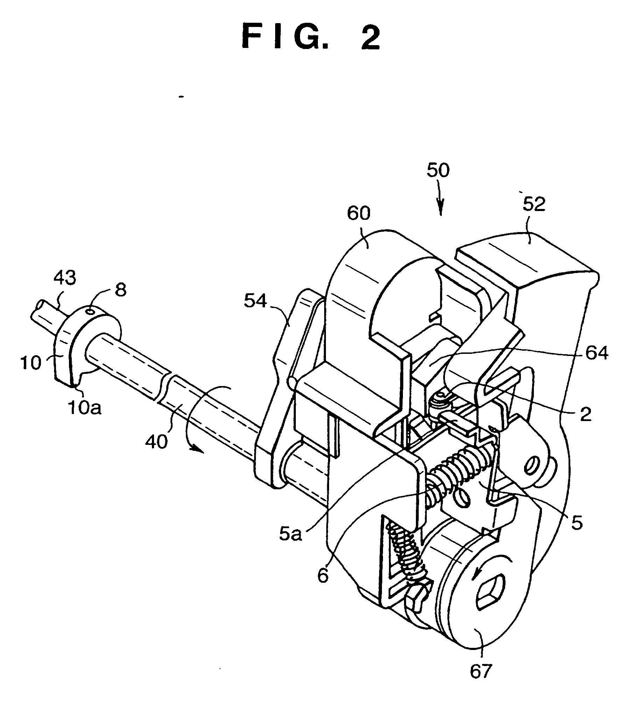

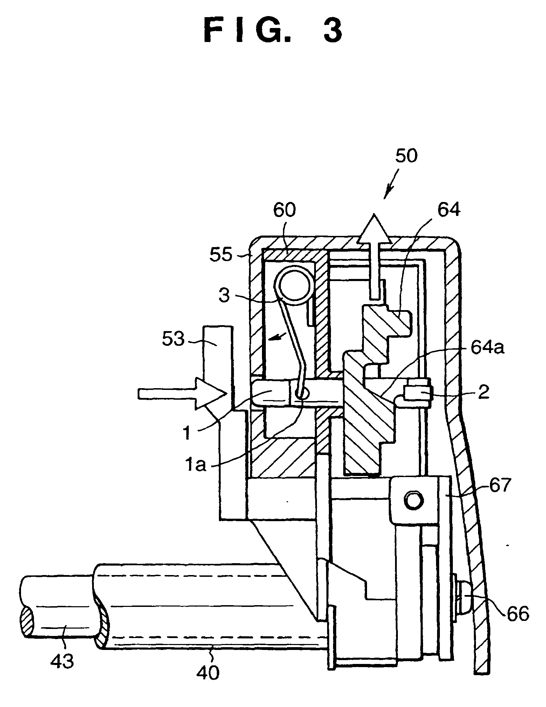

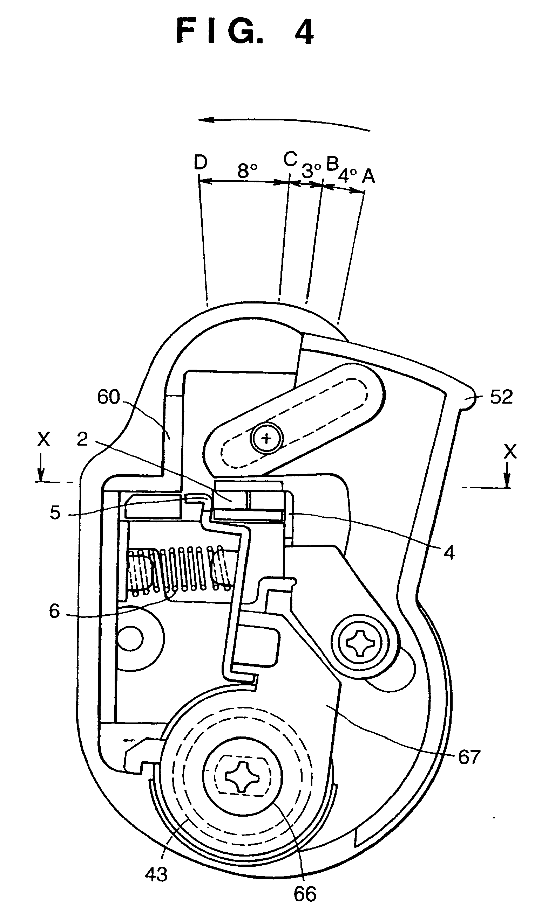

Fig. 2 is a perspective view of the outer appearance of the slider assembly 50 from which a cover is removed, Fig. 3 is a sectional view taken along the line of arrows Y - Y of Fig. 1, and Fig. 4 is a side view. In Figs. 2 to 4, portions that are already described are denoted by the same reference numerals, and a detailed description thereof will be omitted. A tensile spring (not shown) extends above a slider base 60 between the hooks of the clutch lever 52, and biases the left and right hooks 53 and 54 in a normally occlusion state.

The end of the clutch shaft 43 has a locking portion formed by parallel cutting. A plate 67 made of a thick stainless steel plate is fixed to this locking portion with a screw 66. Hence, as the clutch lever 52 pivots, an adjusting bolt fixed to the plate 67 abuts against the end of the clutch shaft 43. The clutch lever 52 is supported to be pivotal about the pipe shaft 40 described above as the center.

A plate cam component 64 has a slant surface 64a, as shown in Fig. 3, and the contact pin 1 is provided to be movable along the slant surface 64a. When the clutch lever 52 is moved to move the plate cam component 64 in a direction of arrow, the left and right hooks 53 and 54 open.

The contact pin 1 extends through the opening of a cover 55 and slides on the plate cam component 64.

More specifically, upon operation of the clutch lever 52, when the hooks 53 and 54 are opened, the motion of the clutch lever 52 is transmitted to the clutch shaft 43 through the plate 67, and the slider assembly 50 is disengaged from a slider feed mechanism 33. Thus, the slider assembly 50 can move in the longitudinal direction.

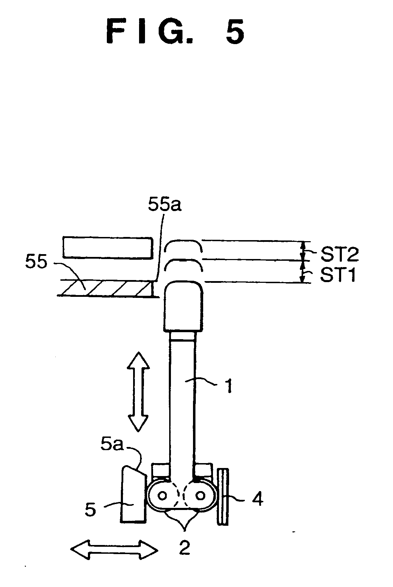

The contact pin 1 axially supports a pair of rollers 2 as shown in Fig. 5 which is the sectional view taken along the line of arrows X - X of Fig. 4. The rollers 2 are sandwiched between a wall surface 4 and a contact pin guide 5. For this purpose, a slant surface 5a and coil spring 6 are provided to the contact pin guide 5. When the contact pin 1 is moved in the direction of a double-headed arrow (vertical directions in Fig. 5), the contact pin guide 5 moves in the direction of double-headed arrow (to the right-to-left direction in Fig. 5).

Since the guide member 5 is fixed to the plate 67, as shown in Fig. 4, it pivots the clutch shaft 43 among positions A, B, C, and D. In the above arrangement, when the flange of the thumb rest of the syringe is not set on the slider assembly 50, the contact pin 1 projects as it is biased by a torsion spring 3. When the user grips the clutch lever 52 to set the flange of the thumb rest, the plate cam component 64 moves upward along a groove formed in the clutch lever 52, and the contact pin 1 is withdrawn inward along the slant surface 64a of the plate cam component 64. Since the plate cam component 64 moves upward, the left and right hooks 53 and 54 are also opened.

Simultaneously with this motion, upon operation of the lever 52, the clutch shaft 43 is pivoted to disengage the half nut member 45 from the lead screw 37, so that the slider assembly 50 can move.

When the user grips thumb rest of the syringe while gripping the clutch lever 52 and then releases the clutch lever 52, the contact pin 1 interferes with the return movement of the contact pin guide 5 as it is pressed by the thumb rest of the syringe. When the thumb rest is not set, the contact pin 1 moves in accordance with the return movement of the clutch lever 52, so the contact pin guide 5 also returns.

The motion of the contact pin guide 5 is transmitted to the operation member 10 through the clutch shaft 43, as described above, and is detected by the photosensor 46, as shown in Figs. 6 and 7.

Disengagement of the syringe is detected in the following manner. After the hooks 53 and 54 are opened upon operation of the clutch lever 52, when the user sets the flange of the thumb rest of the syringe inside the press surface of the cover 55, the contact pin 1 becomes flush with the press surface of the thumb rest. In cooperation with the motion of the contact pin 1, the contact pin guide 5 moves in the right-to-left direction as shown in Fig. 5. The contact pin guide 5 also pivots the dog portion 9b, and this pivot motion is detected by the photosensor 46 serving as a detecting means. If the angle of pivot motion does not fall within a predetermined range (0° to 4°), it is determined that the syringe is not mounted.

In other words, even after the syringe pump is started, presence/absence of the flange of the thumb rest can be monitored by the photosensor 46.

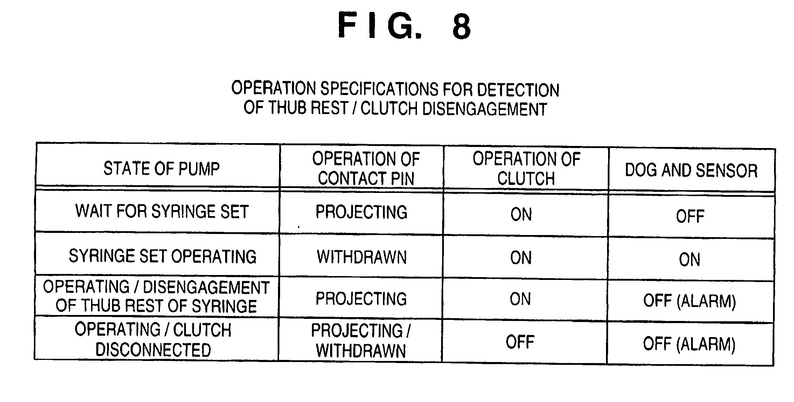

With the above arrangement, disengagement of the flange of the thumb rest of the syringe during operation can be detected, as shown by the operation specification table of Fig. 8. Also, an alarm is produced, if necessary, to warn the user.

As described above, the present invention can provide a syringe pump with which whether the flange of the thumb rest of the syringe is set on the slider assembly of the syringe pump can be detected regardless of whether the pump is in operation or stopped and the flange of the thumb rest can be reliably fixed to the slider assembly, and which is not affected by electric noise.

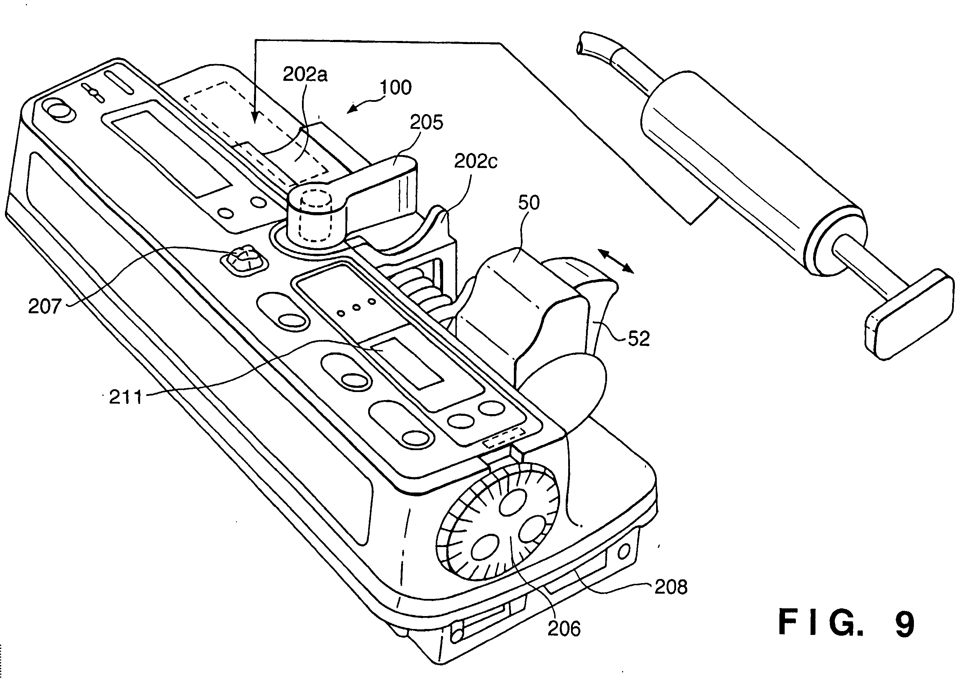

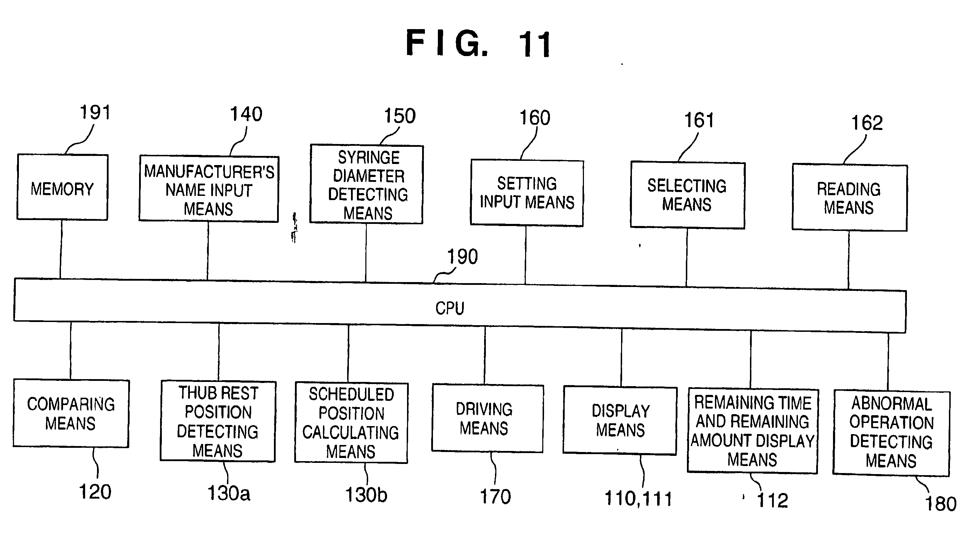

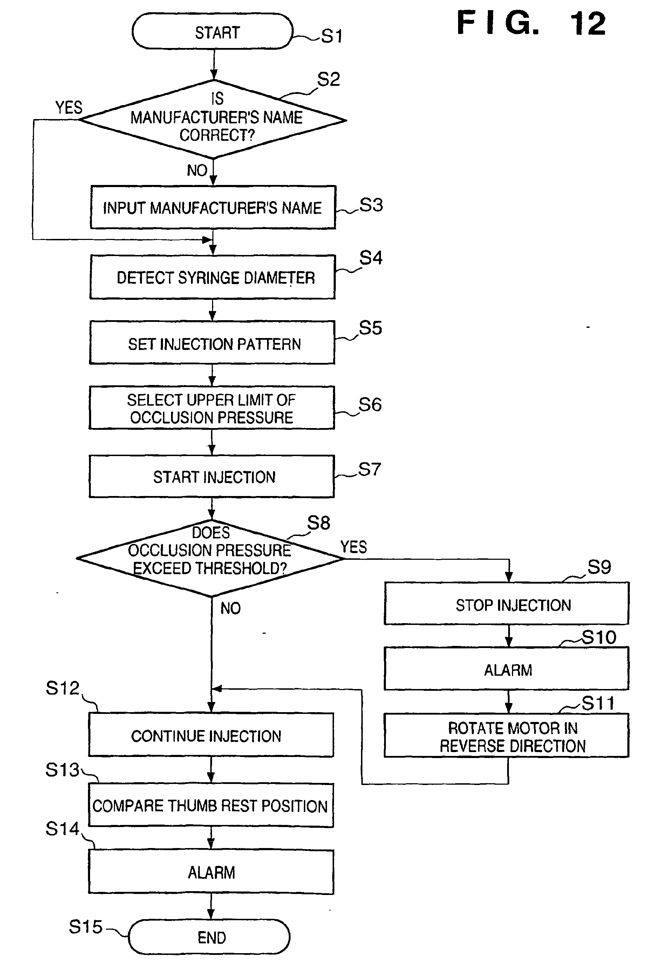

How to use an example of the arrangement of the syringe pump which uses the mechanism of the syringe pump described above will be described with reference to the accompanying drawings. Fig. 9 is a perspective view of the outer appearance of the syringe pump, Fig. 10 is a plan view of an operation panel, Fig. 11 is a block diagram of the syringe pump, and Fig. 12 is a flow chart for explaining the operation of the first embodiment.

<First Embodiment>

As a preparation, the instruments are checked, and whether a syringe pump 100, a pole clamp attached to the syringe pump 100, an AC power cable, a transfusion stand (not shown), a syringe (not shown) containing a liquid drug, and an indwelling needle are all prepared is checked. After that, the pole clamp (not shown) is firmly fixed to the transfusion stand. For this purpose, a pole clamp attaching screw is inserted in a screw hole in the bottom surface of the syringe pump 100, and is set by screwing. The AC power cable is connected to an AC inlet 208 at the right surface of the main body, and the plug is connected to an AC 100 V outlet with a ground terminal.

When the AC power is connected, a battery lamp 217 is turned on to indicate that a built-in battery is being charged.

When the user presses a power switch 215 for about 1 sec to turn on the power supply, all lamps flash three times, and a buzzer sounds to perform self check automatically (step S1) . An AC/DC lamp 216 is turned on, and flow rate, scheduled amount, and integrated amount display lamps 223, 224, and 225 are turned on. A flow rate, scheduled amount, and integrated amount display 211 displays a preset syringe manufacturer's name for about 3 sec in figures. The user must necessarily check whether the manufacturer of the set syringe coincides with the preset manufacturer (step S2).

If the syringe manufacturer is not correct, a correct manufacturer's name is set and input from a manufacture's name input unit (input means) 140 shown in the block diagram of Fig. 11 (step S3) . The set and input manufacturer's name is displayed on the display 211 in characters or figures.

With a lapse of a predetermined period of time, the figures on the flow rate, scheduled amount, and integrated amount display 211 indicating the manufacturer's name disappear, and "0. 0" is displayed on the display 211. An operation indicator 207 is kept off.

A syringe type display lamp 218 displays by flashing all of its four built-in display lamps that a syringe (not shown) is not mounted, to prompt the user to set a syringe. The user sets syringe after all of the above display contents are checked.

An injection line (tube) and a syringe filled with a liquid drug are connected in a sterile atmosphere, and the main body of the syringe is set on a stage 202a shown in Fig. 9 and is locked with a clamp 205.

The user places the flange (not shown) of the syringe in a slit 202c in order to set it there. After that, the user sets the main body of the syringe on the syringe stage 202a, and pivots the clamp 205 in a predetermined direction.

Then, the syringe is unlocked, and is clamped. The syringe diameter (syringe volume) is detected by a syringe diameter detecting means (detector) 150 (step S4).

The user presses a clutch lever 52 of a slider assembly 50 to unlock the clutch, and moves the slider in the directions of arrows. When the clutch lever 52 of the slider assembly 50 is pressed, the slider can be moved manually.

After the thumb rest of the syringe abuts against the slider assembly 50, when the user releases the clutch lever 52, the left and right hooks 53 and 54 automatically hold the thumb rest. In other words, when the user releases the clutch lever 52, the hooks 53 and 54 of the slider assembly 50 sandwich the thumb rest of the syringe.

When the syringe is set, priming is performed. Priming must be necessarily performed before centesis to the patient. When the user presses a fast-forward switch 230, the operation indicator 207 performs rotational display. The pump starts operation and the liquid drug appears at the distal end of the indwelling needle. When the user keeps pressing the fast-forward switch 230 to supply the liquid drug to the distal end of the indwelling needle, the integrated amount lamp 225 flashes.

During fast-forwarding, the integrated amount lamp 225 flashes, and the flow rate, scheduled amount, and integrated amount display 211 displays a priming amount. The priming amount is added to the integrated amount in units of 0.1 m$\ell$ . If the user presses an integration clear switch 227, the integrated amount can be cleared to "zero".

This priming is significant for eliminating the gap between the syringe and the main body so the acting surface of the slider assembly 50 of the main body abuts against the thumb rest of the syringe without any gap, and must accordingly be performed necessarily.

When priming described above is ended, an injection pattern (injection amount) is set at a setting input means (input unit) 160. Prior to setting, whether the flow rate lamp 223 is turned on is checked. If the flow rate lamp 223 is not turned on, the user presses a display selector switch 226 to turn on the flow rate lamp 223.

After that, a setting dial 206 forming part of the input unit 160 is dialed to set a flow rate per hour. In order to prevent erroneous operation of dial setting and to assure safety, the value does not change for a half revolution after the start of dialing. When the setting dial 206 is dialed over a half revolution, the buzzer sounds and the value changes.

The value decreases when the setting dial 206 is dialed counterclockwise, and increases when it is dialed clockwise. While pressing a stop/mute switch 228, when the user dials the setting dial 206, a value at an upper digit position changes rapidly.

Regarding the syringe type and the maximum flow rate, the maximum flow rate that can be set is determined in accordance with the type of the syringe. For example, for a 30 m$\ell$ syringe, the maximum flow rate is 300 m$\ell$/h. When a value larger than the maximum flow rate is set and the start switch 229 is pressed, the flow rate preset value lamp flashes, and injection is not started. Therefore, the injection pattern is set again, and setting operation is completed (step S5).

The occlusion detection pressure level and the buzzer volume level are set by pressing the stop/mute switch 228 and display selector switch 226. To set or change the occlusion detection pressure level, while pressing the stop/mute switch 228, the user presses the display selector switch 226 simultaneously. Then, the flow rate, scheduled amount, and integrated amount display 211 displays "P***", and the setting mode is set. While keeping pressing the stop/mute switch 228, the user releases and presses the display selector switch 226. Then, characters "L" (low), "M" (medium), and "H" (high) printed near occlusion pressure set value lamps 219a, 219b, and 219c are turned on in this order. The user selects a desired occlusion pressure level, releases all the switches, and presses the stop/mute switch 228, and then the integration clear switch 227, thereby selecting, setting, and inputting an occlusion detection pressure level (step S6). "***" mentioned above corresponds to "L, M, and H" described above. In this manner, the stop/mute switch 228, display selector switch 226, and integration clear switch 227 serve as an occlusion pressure selecting means.

On the basis of the selected, set, and input occlusion pressure level, syringe diameter (volume), and syringe manufacturer's name, a corresponding one of occlusion pressure thresholds (upper limits and/or lower limits) which are made up into a table in advance is selected automatically, and injection of the liquid drug is started (step S7).

A predetermined number of data are sampled during a predetermined sampling period (e.g., 16 data are continuously sampled each every 0. 05 sec), the moving average of the data is calculated, and whether the occlusion pressure exceeds the threshold is checked (step S8).

If the occlusion pressure exceeds the threshold, injecting operation is stopped (step S9), and an alarm is produced and displayed (step S10). The motor is rotated for a predetermined amount to return the slider by an amount corresponding to a bolus amount ($V_0(m\ell)$) unique to a predetermined stored cylinder (the motor is rotated in the reverse direction by a predetermined amount), or until reaching a value corresponding to a venous pressure ($F_{IV}(kgf)$) (step S11) .

If the occlusion pressure does not exceed the threshold, injection is continued (step S12). On the basis of data selected and input by a remaining amount/remaining time selection switch (selecting means), a pre-alarm position LNE (cm) is calculated by a controller (CPU) 190 in the following manner.

<Setting with Remaining Amount>

Assume that the pre-alarm position LNE is to be set with a preset remaining amount VNE (mℓ). On the basis of the stored data of the table described above, an alarm position LNE (cm) is calculated as:

$$\text{thumb rest push end position LNE (cm)}$$
$$= \text{LE (cm)} + \text{VNE (m}\ell\text{)}/\text{A (cm}^2\text{)}$$

where LE (cm) is the thumb rest push end position and A (cm$^2$) is the syringe sectional area.

<Setting with Remaining Time>

Assume that the pre-alarm position LNE is to be set with the preset remaining amount VNE (mℓ). On the basis of the stored data of the table described above, the alarm position LNE (cm) is calculated as:

$$\text{thumb rest push end position LNE (cm)}$$
$$= \text{LE (cm)} + \text{R (m}\ell\text{ /h)} \times \text{T (h)}/\text{A (cm}^2\text{)}$$

where R (mℓ /h) is the injection rate, T (h) is the remaining time, and A (cm$^2$) is the syringe sectional area.

Comparative calculation of the pre-alarm position of the thumb rest is performed in the above manner (step S13). When the thumb rest reaches the pre-alarm position, an alarm is produced, and the operation indicator 207 is turned on to emit yellow light rotationally (flashes) (step S14). With a lapse of a predetermined period of time, injection is ended (step S15).

After the syringe is set on the syringe pump 100, if injection is not started with a lapse of about 2 min since flow rate setting operation, the buzzer sounds to inform the user that he might forget to start the operation. When the user presses the stop/mute switch 228 in response to the buzzer, the buzzer stops. If the user wishes to change the injection pattern (flow rate) during injection, he performs temporary stop of injection to stop injection, and sets the injection pattern again with the setting input means 160 including the setting dial 206. At this time, when the user presses the stop/mute switch 228, the operation indicator 207 is turned off.

When a scheduled amount of liquid drug is injected, the user checks the stop state and removes the syringe. For this purpose, the user pulls up the clamp 205, rotates it through about 90°, and holds it in this state. The user then presses the clutch lever 52 of the slider assembly 50 to open left and right hooks 53 and 54, and removes the syringe. After that, if the user will not use the syringe pump again, he presses the power switch 215 for about 2 sec or more to turn it off.

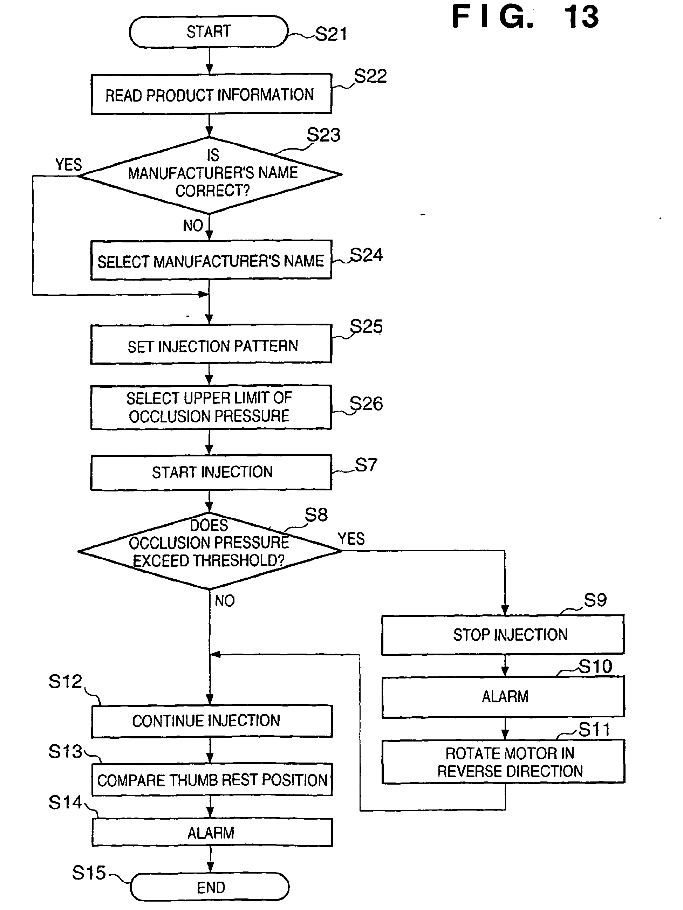

A case wherein product information adhered to the syringe is to be automatically read by a reading means 162 will be described with reference to the operation flow chart of Fig. 13 and the block diagram of Fig. 11.

When the user presses the power switch 215 for about 1 sec to turn on the power supply, all lamps flash three times, and a buzzer sounds to perform self check automatically (step S21). The AC/DC lamp 216 is turned on, and the flow rate, scheduled amount, and integrated amount display lamps 223, 224, and 225 are turned on. The flow rate, scheduled amount, and integrated amount display 211 displays a preset syringe manufacturer's name for about 3 sec in figures. The product information (bar code) of the set syringe is automatically read by the product information reading means 162 to identify the type of syringe (step S22). The user must necessarily check whether the manufacturer of the set syringe coincides with the preset manufacturer (step S23).

If the syringe manufacturer is different from the preset one, a correct manufacturer's name is set and input from the manufacture's name input unit (input means) 140 (step S24) . If the syringe manufacturer is identical to the preset one, the next injection pattern is set (step S25). The set and input manufacturer's name is displayed on the display 211 in characters or figures.

When the injection pattern is set and input, an occlusion pressure threshold is set (step S26). How to use the syringe pump after this is identical to that of the operation flow chart of Fig. 12, and a detailed description thereof will be omitted.

<Second Embodiment>

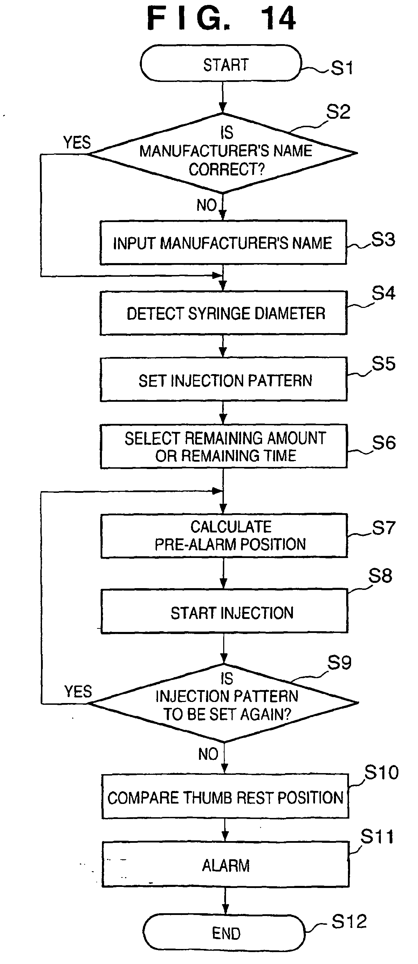

Fig. 14 is a flow chart for explaining the operation of the second embodiment. In Fig. 14, steps that are identical to those already described with reference to the operation flow chart of Fig. 12 will be denoted by the same reference numerals as in Fig. 12, and a detailed description thereof will be omitted.

When the injection pattern is set in step S5, the flow advances to step S6, and the user selects and inputs whether an alarm is to be produced when the liquid drug in the syringe has reached a predetermined remaining amount or when the thumb rest of the syringe has reached a predetermined position with the remaining amount/remaining time selection switch of a selecting means 161. On the basis of the selected input data and the preset input value of the injection pattern of the flow rate, the pre-alarm position LNE (cm) is calculated by a controller 190 in the following manner (step S7).

<Setting with Remaining Amount>

Assume that the pre-alarm position LNE is to be set with a preset remaining amount VNE (m$\ell$). On the basis of the stored data of the table described above, an alarm position LNE (cm) is calculated as: thumb rest push end position LNE (cm) = LE (cm) + VNE (m$\ell$)/A (cm$^2$) where LE (cm) is the thumb rest push end position.

<Setting with Remaining Time>

Assume the pre-alarm position LNE is to be set with a preset remaining amount VNE (m$\ell$). On the basis of the stored data of the table described above, the alarm position LNE (cm) is calculated as: thumb rest push end position LNE (cm) = LE (cm) + R (m$\ell$ /h) x T (h) /A (cm$^2$) where R (m$\ell$ /h) is the injection speed, T (h) is the remaining time, and A (cm$^2$) is the syringe sectional area.

When the injection pattern is set in the above manner, the user checks whether the main body 1 is in the stop state, and punctures the patient's arm or the like with an indwelling needle. When all the operations are completed, the liquid drug is injected. When the user presses a start switch 229 for this purpose, injection is started (step S8). Since an operation indicator 207 is turned on rotationally, the user can check the injection state of the liquid drug at a place far from the syringe pump.

After the syringe is set on a main body 100, if injection is not started with a lapse of about 2 min since flow rate setting operation, the buzzer sounds to inform the user that he might forget to start the operation. When the user presses a stop/mute switch 228 in response to the buzzer, the buzzer stops. If the user wishes to change the flow rate injection pattern during injection, he performs temporary stop of injection to stop injection, and sets the injection pattern again with a setting input means 160 including a setting dial 206 (step S9). At this time, when the user presses the stop/mute switch 228, the operation indicator 207 is turned off.

After injection is started, the user checks the integrated amount. For this purpose, the user presses a display selector switch 226 to turn on an integration amount lamp 225. Then, a flow rate, scheduled amount, and integrated amount display 211 displays an integrated amount since the start of injection for about 15 sec in units of 0.1 m$\ell$.

To clear the integrated amount, the user presses the stop/mute switch 228 to stop the main body 100, the display selector switch 226 to display the integrated amount, and then an integration clear switch 227 for about 1.5 sec. The buzzer sounds, and the integrated amount of the flow rate, scheduled amount, and integrated amount display 211 is cleared to "0.01 m$\ell$". A thumb rest position detected by a thumb rest position detecting means (detection unit) 130a including a potentiometer is compared with the pre-alarm position by a comparing means 120 (step S10). If it is determined that the thumb rest has reached the pre-alarm position, the operation indicator 207 makes an alarm (step S11).

When a scheduled amount of liquid drug is injected, the user removes the syringe in step S12. For this purpose, the user pulls up the clamp 205, rotates it through about 90°, and holds it in this state. The user then presses a clutch lever 52 of a slider assembly 50 to open left and right hooks 53 and 54, and removes the syringe. After that, if the user will not use the syringe pump again, he presses a power switch 215 for about 2 sec or more to turn it off.

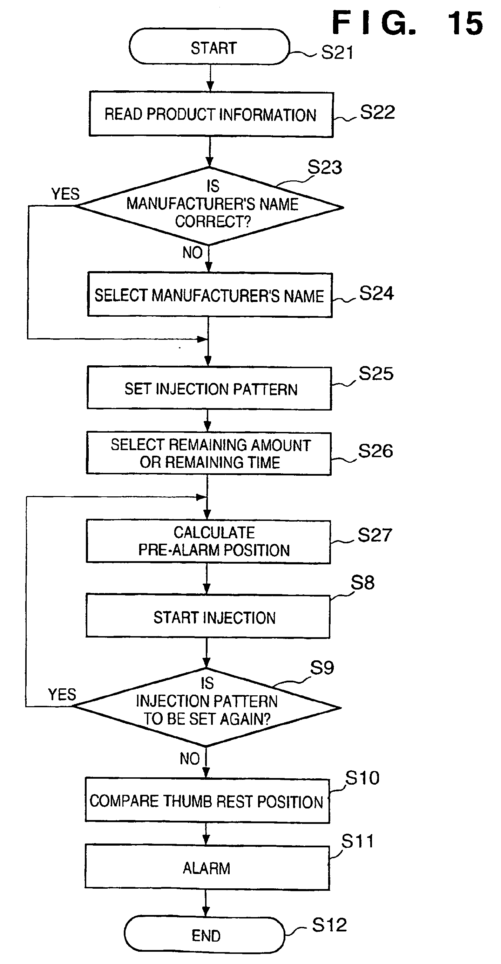

A case wherein product information adhered to the syringe is to be automatically read by a reading means 162 will be described with reference to the operation flow chart of Fig. 15 and the block diagram of Fig. 11.

When the user presses the power switch 215 for about 1 sec to turn on the power supply, all lamps flash three times, and a buzzer sounds again to perform self check automatically (step S21). An AC/DC lamp 216 is turned on, and flow rate and scheduled amount display lamps 223 and 224 and the integrated amount display lamp 225 are turned on. The flow rate, scheduled amount, and integrated amount display 211 displays a preset syringe manufacturer's name for about 3 sec in figures. The product information (such as bar code) of the set syringe is automatically read by the product information reading means 162 to specify the type of syringe (step S22). The user must necessarily check whether the manufacturer of the set syringe coincides with the preset manufacturer's name (step S23).

If the syringe manufacturer is different from the preset one, a correct manufacturer's name is set and input from a manufacture's name input unit (input means) 140 (step S24). If the syringe manufacturer is identical to the preset one, the next injection pattern is set (step S25). The set and input manufacturer's name is displayed on the display 211 in characters or figures. If the syringe manufacturer's name is correct, the injection pattern is set and input in step S25.

When the injection pattern is set and input, the user selects and inputs whether an alarm is to be produced when the liquid drug in the syringe has reached a predetermined remaining amount or when the thumb rest of the syringe has reached a predetermined position with the remaining amount and remaining time selection switch of the selecting means 161 (step S26).

<Setting with Remaining Amount>

Assume that the pre-alarm position LNE is to be set with a preset remaining amount VNE (mℓ). On the basis of the stored data of the table described above, an alarm position LNE (cm) is calculated as:

$$\texttt{thumb rest push end position LNE (cm)}$$
$$\texttt{= LE (cm) + VNE (m}\ell\texttt{)/A (cm}^2\texttt{)}$$

where LE (cm) is the thumb rest push end position and A (cm$^2$) is the syringe sectional area.

<Setting with Remaining Time>

Assume that the pre-alarm position LNE is to be set with a preset remaining amount VNE (mℓ). On the basis of the stored data of the table described above, the alarm position LNE (cm) is calculated as:

$$\texttt{thumb rest push end position LNE (cm)}$$
$$\texttt{= LE (cm) + R (m}\ell\texttt{/h) x T (h)/A (cm}^2\texttt{)}$$

where R (mℓ /h) is the injection speed, T (h) is the remaining time, and A (cm$^2$) is the syringe sectional area.

The flow then advances to step S27 corresponding to step S7 of Fig. 14, steps S8, S9, S10, S11, and S12 similar to those Fig. 14 are performed, and the flow is ended.

The syringe pump with the above functions can sufficiently achieve desired functions. More effective liquid drug injection can be performed with an injection setting function per weight, e.g., μg/kg/min, mg/kg/h, or the like.

<Third Embodiment>

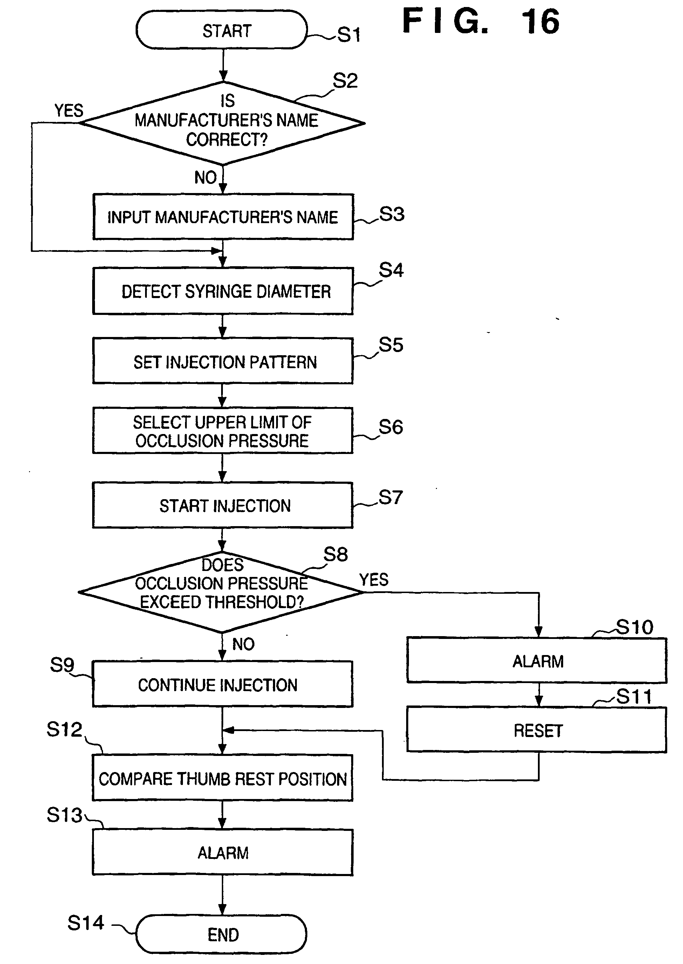

Fig. 16 is a flow chart for explaining the operation of the third embodiment. In Fig. 16, steps that are identical to those already described with reference to the operation flow chart of Fig. 12 will be denoted by the same reference numerals as in Fig. 12, and a detailed description thereof will be omitted.

In Fig. 16, steps S1 to S7 are identical to those of the operation flow chart of Fig. 12.

In step S8, a predetermined number of data are sampled during a predetermined sampling period (e.g., 16 data are continuously sampled each every 0.05 sec), the moving average of the data is calculated, and whether the occlusion pressure exceeds the threshold is checked (step S8).

If the occlusion pressure exceeds the threshold, an alarm is produced, and an operation indicator 207 is turned on or flashes in red to inform the alarm to the user (step S10) . The user checks the occlusion pressure. If there is no problem, the syringe pump is reset (step S11).

Steps S12, S13, and S14 are performed, and the flow is ended.

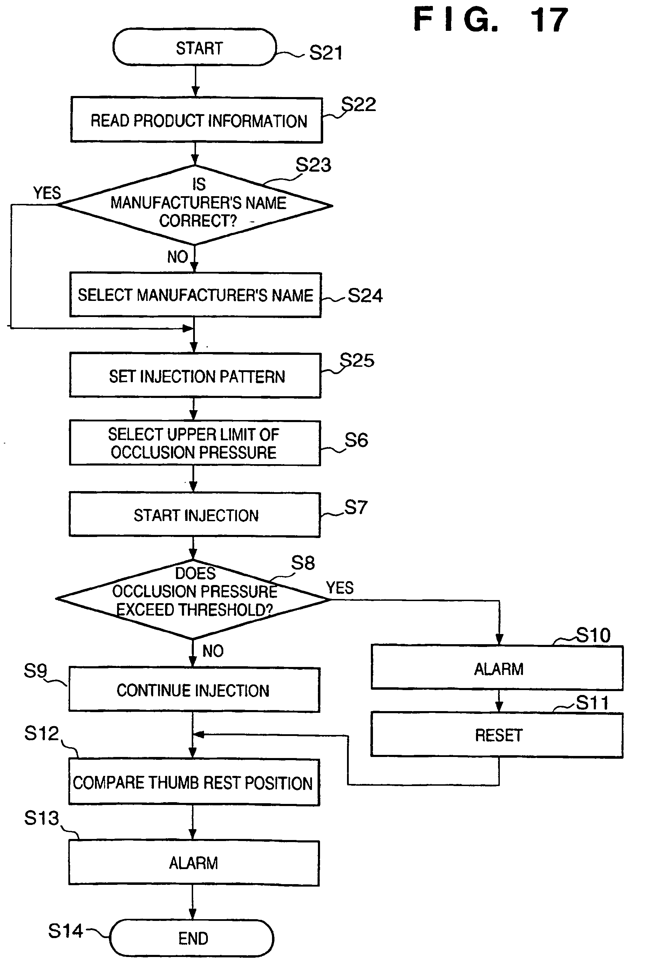

Finally, a case wherein product information adhered to the syringe is to be automatically read by a reading means 162 will be described with reference to the operation flow chart of Fig. 17 and the block diagram of Fig. 11.

When the user presses a power switch 215 for about 1 sec to turn on the power supply, all lamps flash three times, and a buzzer sounds again to perform self check automatically (step S21). An AC/DC lamp 216 is turned on, and flow rate, scheduled amount, and integrated amount display lamps 223, 224, and 225 are turned on. A flow rate, scheduled amount, and integrated amount display 211 displays a preset syringe manufacturer's name for about 3 sec in figures. The product information (bar code) of the set syringe is automatically read by the product information reading means 162 to identify the type of syringe (step S22). The user must necessarily check whether the manufacturer of the set syringe coincides with the preset manufacturer (step S23).

If the syringe manufacturer is different from the preset one, a correct manufacturer's name is set and input from a manufacture's name input unit (input means) 140 (step S24). If the syringe manufacturer is identical to the preset one, the next injection pattern is set (step S25). The set and input manufacturer's name is displayed on the display 211 in characters or figures.

When the injection pattern is set and input, an occlusion pressure threshold is set (step S26). How to use the syringe pump after this is identical to that of the operation flow chart of Fig. 16, and a detailed description thereof will be omitted.

With the syringe pump described above, occlusion detection can be performed more accurately with various types of injection patterns (program input patterns) and various types of syringes, and drive control of the syringe can be performed without causing bolus, even when an occlusion is detected.

An alarm can be produced a predetermined period of time (hours, minutes) before scheduled injection end time, based on a predetermined liquid drug remaining amount in the syringe and/or on a predetermined position of the thumb rest of the syringe before liquid drug injection is ended, and not on the various types of injection patterns (program input patterns). A syringe pump can be obtained which can arbitrarily select an occlusion pressure for various types of syringes in accordance with syringe volumes (syringe diameters) and syringe manufacturer or for a syringe containing a predetermined liquid drug, so that it can detect an occlusion pressure precisely.

**Claims**

1. A syringe pump for detachably holding a syringe main body on an apparatus main body in an immobile state, setting a flange of a thumb rest of the syringe on slider means in an immobile state, and driving said slider means, thereby accurately feeding out a content in the syringe,

   said slider means comprising
   a lead screw (37) axially supported by a base portion (34) so as to be driven rotationally,
   a hollow shaft (40) provided to said base portion to be extendable in a longitudinal direction of the syringe,
   a clutch shaft (43) rotatably provided in a hollow portion of said hollow shaft (40),
   a half nut member (45) provided to one end of said clutch shaft (43) and positioned in a meshing state to mesh with a thread portion (37a) of said lead screw and in a disengaged state disengaged from said thread portion,
   a detector with dog means (39b) to abut against said half nut member (45) and block light to a sensor (46) fixed to said base portion in order to detect that said half nut member (45) is in the meshing state,
   a movable base portion (60) fixed to the other end of said hollow shaft,
   said movable base portion (60) being comprised of a pair of hooks (53, 54) for setting the flange of the thumb rest in the immobile state,
   **characterized in that** said movable base portion comprises converting means (2, 4, 5) for converting an abutting state against the thumb rest of the flange into rotation of said clutch shaft (43) through a contact pin (1), and
   a power transmission mechanism (7, 9, 10) is provided for transmitting a moving amount of said contact pin to said half nut member (45) before said detector detects that said half nut member is in the meshing state upon movement of said dog means,
   wherein said detector can detect with said detector that the flange of the thumb rest has disengaged from said hooks.

2. The syringe pump according to claim 1, **characterized in that** said power transmission mechanism comprises:

   an operation member (10) fixed to said one end of said clutch shaft (43) and having a projection (10a), said half nut member (45) pivotally provided to said clutch shaft (43),
   a stud (7) fixed to said half nut member (45) at a predetermined gap from said projection of said operation member (10), and
   an intermediate member (9) having an abutting portion (9a) with one end abutting against a shaft (39) of said dog means (39D) pivotally provided to said base portion, a hole portion pivotally, axially supported by said clutch shaft, and a portion (9b) with a spring (15) which biases said operation member to separate from said stud by a distance corresponding to a clearance.

3. The syringe pump according to claim 1 or 2, **characterized in that** said hooks are opened and said contact pin moves inward upon operation of a clutch lever (52) provided to said movable base portion.

**Patentansprüche**

1. Spritzenpumpe zum lösbaren Halten eines Spritzenhauptkörpers auf einem Vorrichtungshauptkörper im unbeweglichen Zustand, zum Setzen eines Flansches einer Daumenauflage der Spritze auf eine Schiebeeinrichtung im unbeweglichen Zustand und zum Antrieb der Schiebeeinrichtung, wodurch ein Inhalt der Spritze präzise ausgebracht wird,

   wobei die Schiebeeinrichtung aufweist:

   eine Gewindespindel (37), die axial von einem Basisabschnitt (34) so gestützt wird, dass sie drehend angetrieben wird,
   eine Hohlwelle (40), die an dem Basisabschnitt so vorgesehen ist, dass sie sich in Längsrichtung der Spritze erstrecken kann,
   eine Kupplungswelle (43), die in einem hohlen Abschnitt der Hohlwelle (40) drehbar vorgesehen ist, ein Mutterschlosselement (45), das an einem Ende der Kupplungswelle (43) vorgesehen und im Eingriffszustand so positioniert ist, dass es in einen Gewindeabschnitt (37a) der Gewindespindel eingreift und im ausgerückten Zustand von dem Gewindeabschnitt gelöst ist,
   einen Detektor mit einer Anschlageinrichtung (39b) zum Anschlag gegen das Mutterschlosselement (45) und zum Abblocken von Licht von einem Sensor (46), der an dem Basisabschnitt befestigt ist, um zu ermitteln, dass sich das Mutterschlosselement (45) im Eingriffszustand befindet,
   einem beweglichen Basisabschnitt (60), der am anderen Ende der Hohlwelle befestigt ist,
   wobei der bewegliche Basisabschnitt (60) aus einem Paar Haken (53, 54) zum Setzen des Flansches der Daumenauflage im unbeweglichen Zustand besteht,
   **dadurch gekennzeichnet,**
   **dass** der bewegliche Basisabschnitt aufweist:

   Umwandlungseinrichtungen (2, 4, 5) zum Umwandeln des Anschlagszustands gegen die Daumenauflage des Flansches in eine Drehung der Kupplungswelle (43) durch einen Kontaktstift (1), und
   **dass** ein Kraftübertragungsmechanismus (7, 9, 10) vorgesehen ist zum Übertragen eines Bewegungsbetrags des Kontaktstifts auf das Mutterschlosselement (45) bevor der Detektor ermittelt, dass sich das Mutterschlosselement nach der Bewegung der Anschlageinrichtung im Eingriffszustand befindet, wobei der Detektor ermitteln kann, dass sich der Flansch der Daumenauflage von den Haken gelöst hat.

2. Spritzenpumpe nach Anspruch 1, **dadurch gekennzeichnet, dass** der Kraftübertragungsmechanismus aufweist:

   ein Betätigungselement (10), das an dem einen Ende der Kupplungswelle (43) befestigt ist und einen Vorsprung (10a) aufweist, wobei das Mutterschlosselement (45) schwenkbar an der Kupplungswelle (43) vorgesehen ist, einen Zapfen (7), der an dem Mutterschlosselement (45) in einem vorgegebenen Abstand von dem Vorsprung des Betätigungselements (10) befestigt ist, und
   einem Zwischenelement (9) mit einem Anschlagsabschnitt (9a), wobei ein Ende an einer Welle (39) des Anschlagselements (39D) anschlägt, das schwenkbar an dem Basisabschnitt vorgesehen ist, einem Lochabschnitt, der schwenkbar axial von der Kupplungswelle gestützt wird, und einen Abschnitt (9b) mit einer Feder (15), die das Betätigungselement so vorspannt, dass es von dem Zapfen um einen Abstand, der einem Spiel entspricht, getrennt wird.

3. Spritzenpumpe nach Anspruch 1 oder 2, **dadurch gekennzeichnet, dass** die Haken geöffnet sind und der Kontaktstift sich bei Betätigung eines Kupplungshebels (52), der an dem beweglichen Basisabschnitt vorgesehen ist, nach innen bewegt.

**Revendications**

1. Pompe de seringue pour tenir de manière détachable un corps principal de seringue sur un corps principal de dispositif à l'état immobile, installer une bride d'un appui pour le pouce de la seringue sur des moyens coulissants à l'état immobile, et entraîner lesdits moyens coulissants, fournissant de ce fait de manière précise un contenu de la seringue,

   lesdits moyens coulissants comprenant
   une vis mère (37) supportée de manière axiale par une partie de base (34) de façon à être entraînée en rotation,
   un arbre creux (40) fourni à ladite partie de base de façon à pouvoir s'étendre suivant une direction longitudinale de la seringue,

un arbre d'accouplement (43) fourni de manière rotative dans une partie creuse dudit arbre creux (40),

un élément de demi-écrou (45) fourni au niveau d'une extrémité dudit arbre d'accouplement (43) et positionné dans un état en engrènement de façon à entrer en engrènement avec une partie filetée (37a) de ladite vis-mère et dans un état désengagé où il est désengagé de ladite partie filetée,

un détecteur avec des moyens de cliquets (39b) de façon à entrer en butée contre ledit élément de demi-écrou (45) et bloquer la lumière reçue par un capteur (46) fixé à ladite partie de base afin de détecter que ledit élément de demi-écrou (45) est à l'état d'engrènement,

Une partie de base mobile (60) fixée à l'autre extrémité dudit arbre creux,

ladite partie de base mobile (60) étant constituée d'une paire de crochets (53, 54) pour installer la bride de l'appui pour le pouce à l'état immobile,

**caractérisée en ce que** ladite partie de base mobile comprend des moyens de conversion (2, 4, 5) pour convertir un état en butée contre l'appui pour le pouce de la bride en une rotation dudit arbre d'accouplement (43) par l'intermédiaire d'une broche de contact (1), et

un dispositif de transmission de puissance (7, 9, 10) est fourni pour transmettre une quantité de déplacement de ladite broche de contact au dit élément de demi-écrou (45) avant que ledit détecteur ne détecte que ledit élément de demi-écrou est à l'état d'engrènement lors du mouvement desdits moyens de cliquets,

dans laquelle ledit détecteur peut détecter avec ledit détecteur que la bride de l'appui pour le pouce a été désengagée desdits crochets.

**2.** Pompe de seringue selon la revendication 1, **caractérisée en ce que** ledit dispositif de transmission de puissance comprend :

un élément fonctionnel (10) fixé à ladite une extrémité dudit arbre d'accouplement (43) et ayant une saillie (10a),
ledit élément de demi-écrou (45) étant fourni de manière pivotante au dit arbre d'accouplement (43),
un goujon (7) fixé au dit élément de demi-écrou (45) à un espace prédéterminé de ladite saillie dudit élément fonctionnel (10), et
un élément intermédiaire (9) ayant une partie de butée (9a) avec une extrémité entrant en butée contre un arbre (39) desdits moyens de cliquets (39D) fourni de manière pivotante à ladite partie de base, une partie d'orifice supportée par ledit arbre d'accouplement de manière axiale et pivotante, et une partie (9b) avec un ressort (15) qui dévie ledit élément fonctionnel de façon à le séparer dudit goujon selon une distance qui correspond à un espace libre.

**3.** Pompe de seringue selon la revendication 1 ou la revendication 2, **caractérisée en ce que** lesdits crochets sont ouverts et ladite broche de contact se déplace à l'activation d'un levier d'accouplement (52) fourni à ladite partie de base mobile.

F I G. 1

# F I G. 2

# FIG. 3

# F I G. 4

# F I G. 5

# F I G. 6

# F I G. 7

# FIG. 8

OPERATION SPECIFICATIONS FOR DETECTION
OF THUB REST / CLUTCH DISENGAGEMENT

| STATE OF PUMP | OPERATION OF CONTACT PIN | OPERATION OF CLUTCH | DOG AND SENSOR |
|---|---|---|---|
| WAIT FOR SYRINGE SET | PROJECTING | ON | OFF |
| SYRINGE SET OPERATING | WITHDRAWN | ON | ON |
| OPERATING / DISENGAGEMENT OF THUB REST OF SYRINGE | PROJECTING | ON | OFF (ALARM) |
| OPERATING / CLUTCH DISCONNECTED | PROJECTING / WITHDRAWN | OFF | OFF (ALARM) |

EP 1 110 569 B1

**F I G. 9**

EP 1 110 569 B1

FIG. 10

# FIG. 11

MEMORY 191

MANUFACTURER'S NAME INPUT MEANS 140

SYRINGE DIAMETER DETECTING MEANS 150

SETTING INPUT MEANS 160

SELECTING MEANS 161

READING MEANS 162

CPU 190

COMPARING MEANS 120

THUB REST POSITION DETECTING MEANS 130a

SCHEDULED POSITION CALCULATING MEANS 130b

DRIVING MEANS 170

DISPLAY MEANS 110,111

REMAINING TIME AND REMAINING AMOUNT DISPLAY MEANS 112

ABNORMAL OPERATION DETECTING MEANS 180

EP 1 110 569 B1

**F I G. 12**

```
                    ┌─────────────┐
                    │    START    │──S1
                    └─────────────┘
                           │
                           ▼
                    ╱ ╲ S2
         YES    ╱    IS    ╲
      ┌────────╱ MANUFACTURER'S╲
      │        ╲    NAME    ╱
      │          ╲ CORRECT?╱
      │            ╲ ╱
      │          NO │
      │             ▼
      │   ┌──────────────────────────┐
      │   │ INPUT MANUFACTURER'S NAME │──S3
      │   └──────────────────────────┘
      │             │
      └─────────────┤
                    ▼
          ┌──────────────────────┐
          │ DETECT SYRINGE DIAMETER │──S4
          └──────────────────────┘
                    │
                    ▼
          ┌──────────────────────┐
          │  SET INJECTION PATTERN │──S5
          └──────────────────────┘
                    │
                    ▼
          ┌──────────────────────┐
          │  SELECT UPPER LIMIT OF │──S6
          │  OCCLUSION PRESSURE    │
          └──────────────────────┘
                    │
                    ▼
          ┌──────────────────────┐
          │    START INJECTION    │──S7
          └──────────────────────┘
                    │
                    ▼
                 ╱ ╲ S8
              ╱ DOES ╲
           ╱ OCCLUSION ╲  YES            ┌──────────────────┐
          ╱  PRESSURE    ╲───────────────│  STOP INJECTION  │──S9
          ╲EXCEED THRESHOLD?╱            └──────────────────┘
           ╲    ╱                                 │
            ╲ ╱                                   ▼
          NO │                          ┌──────────────────┐
             │                          │      ALARM       │──S10
             │                          └──────────────────┘
             │                                   │
             ▼                                   ▼
   S12 ┌──────────────────┐          ┌──────────────────────┐
       │ CONTINUE INJECTION│◄─────┐   │ ROTATE MOTOR IN      │──S11
       └──────────────────┘      │   │ REVERSE DIRECTION    │
             │                   │   └──────────────────────┘
             ▼                   │            │
   S13 ┌──────────────────────┐  └────────────┘
       │ COMPARE THUMB REST POSITION │
       └──────────────────────┘
             │
             ▼
   S14 ┌──────────────────┐
       │      ALARM       │
       └──────────────────┘
             │
             ▼
   S15 ┌──────────────────┐
       │       END        │
       └──────────────────┘
```

**F I G. 13**

START ～S21

READ PRODUCT INFORMATION ～S22

IS MANUFACTURER'S NAME CORRECT? S23
YES / NO

SELECT MANUFACTURER'S NAME ～S24

SET INJECTION PATTERN ～S25

SELECT UPPER LIMIT OF OCCLUSION PRESSURE S26

START INJECTION ～S7

DOES OCCLUSION PRESSURE EXCEED THRESHOLD? S8
NO / YES

STOP INJECTION S9

ALARM S10

ROTATE MOTOR IN REVERSE DIRECTION S11

S12 CONTINUE INJECTION

S13 COMPARE THUMB REST POSITION

S14 ALARM

S15 END

# F I G. 14

```
        ┌─────────────┐
        │    START    │ ──S1
        └──────┬──────┘
               │
               ▼
          ╱─────────╲
   YES   ╱    IS     ╲ ──S2
  ◄─────╱ MANUFACTURER'S╲
        ╲   NAME       ╱
         ╲  CORRECT?  ╱
          ╲─────────╱
               │ NO
               ▼
    ┌──────────────────────┐
    │ INPUT MANUFACTURER'S │ ──S3
    │        NAME          │
    └──────────┬───────────┘
               │
               ▼
    ┌──────────────────────┐
    │ DETECT SYRINGE DIAMETER │ ──S4
    └──────────┬───────────┘
               │
               ▼
    ┌──────────────────────┐
    │ SET INJECTION PATTERN │ ──S5
    └──────────┬───────────┘
               │
               ▼
    ┌──────────────────────┐
    │ SELECT REMAINING AMOUNT │ ──S6
    │    OR REMAINING TIME    │
    └──────────┬───────────┘
               │
               ▼
    ┌──────────────────────┐
    │      CALCULATE        │ ──S7
    │  PRE-ALARM POSITION   │
    └──────────┬───────────┘
               │
               ▼
    ┌──────────────────────┐
    │    START INJECTION    │ ──S8
    └──────────┬───────────┘
               │
               ▼
          ╱─────────╲
   YES   ╱    IS     ╲ ──S9
  ◄─────╱ INJECTION PATTERN ╲
        ╲ TO BE SET AGAIN? ╱
          ╲─────────╱
               │ NO
               ▼
    ┌──────────────────────┐
    │ COMPARE THUMB REST POSITION │ ──S10
    └──────────┬───────────┘
               │
               ▼
    ┌──────────────────────┐
    │        ALARM         │ ──S11
    └──────────┬───────────┘
               │
               ▼
        ┌─────────────┐
        │     END     │ ──S12
        └─────────────┘
```

# FIG. 15

```
        ┌──────────────────┐
        │      START       │──S21
        └──────────────────┘
                 │
        ┌──────────────────────────┐
        │ READ PRODUCT INFORMATION │──S22
        └──────────────────────────┘
                 │
                 ▼           S23
              ╱         ╲
            ╱     IS      ╲
   YES ───╱ MANUFACTURER'S ╲
          ╲     NAME        ╱
            ╲  CORRECT?   ╱
              ╲         ╱
                 │ NO
        ┌──────────────────────────┐
        │ SELECT MANUFACTURER'S NAME│──S24
        └──────────────────────────┘
                 │
        ┌──────────────────────────┐
        │   SET INJECTION PATTERN  │──S25
        └──────────────────────────┘
                 │
        ┌──────────────────────────┐
        │  SELECT REMAINING AMOUNT │──S26
        │     OR REMAINING TIME    │
        └──────────────────────────┘
                 │
        ┌──────────────────────────┐
        │       CALCULATE          │──S27
        │   PRE-ALARM POSITION     │
        └──────────────────────────┘
                 │
        ┌──────────────────────────┐
        │     START INJECTION      │──S8
        └──────────────────────────┘
                 │
                 ▼           S9
              ╱         ╲
            ╱     IS      ╲
   YES ───╱ INJECTION PATTERN╲
          ╲  TO BE SET AGAIN? ╱
              ╲         ╱
                 │ NO
        ┌──────────────────────────┐
        │COMPARE THUMB REST POSITION│──S10
        └──────────────────────────┘
                 │
        ┌──────────────────────────┐
        │          ALARM           │──S11
        └──────────────────────────┘
                 │
        ┌──────────────────┐
        │       END        │──S12
        └──────────────────┘
```

# FIG. 16

START — S1

S2
IS MANUFACTURER'S NAME CORRECT?

YES

NO

INPUT MANUFACTURER'S NAME — S3

DETECT SYRINGE DIAMETER — S4

SET INJECTION PATTERN — S5

SELECT UPPER LIMIT OF OCCLUSION PRESSURE — S6

START INJECTION — S7

S8
DOES OCCLUSION PRESSURE EXCEED THRESHOLD?

YES

NO

S9 — CONTINUE INJECTION

S10 — ALARM

S11 — RESET

S12 — COMPARE THUMB REST POSITION

S13 — ALARM

S14 — END

# F I G. 17

```
          ┌──────────────┐
          (    START     )──── S21
          └──────┬───────┘
          ┌──────▼───────────────┐
          │ READ PRODUCT INFORMATION │──── S22
          └──────┬───────────────┘
                 ▼              S23
              ◇ IS ◇
YES   ◇ MANUFACTURER'S NAME ◇
      ◇   CORRECT?   ◇
                 │ NO
          ┌──────▼───────────────┐
          │ SELECT MANUFACTURER'S NAME │──── S24
          └──────────────────────┘

          ┌──────────────────────┐
          │  SET INJECTION PATTERN │──── S25
          └──────┬───────────────┘
          ┌──────▼───────────────┐
          │ SELECT UPPER LIMIT OF  │──── S6
          │  OCCLUSION PRESSURE    │
          └──────┬───────────────┘
          ┌──────▼───────────────┐
          │    START INJECTION     │──── S7
          └──────┬───────────────┘
                 ▼           S8
         ◇ DOES ◇            YES
      ◇ OCCLUSION PRESSURE ◇──────────┐
      ◇ EXCEED THRESHOLD? ◇           │
                 │ NO              ┌───▼────┐
S9 ┌─────────────▼──────┐          │ ALARM  │── S10
   │  CONTINUE INJECTION │          └───┬────┘
   └─────────────┬──────┘          ┌───▼────┐
S12              │                 │ RESET  │── S11
   ┌─────────────▼──────────────┐  └───┬────┘
   │ COMPARE THUMB REST POSITION │◄────┘
   └─────────────┬──────────────┘
S13 ┌────────────▼──────┐
    │      ALARM         │
    └────────────┬──────┘
S14 ┌────────────▼──────┐
    (      END          )
    └───────────────────┘
```

# FIG. 18

# F I G. 19